(19) Europäisches Patentamt
European Patent Office
Office européen des brevets

(11) **EP 2 474 588 A2**

(12) **EUROPEAN PATENT APPLICATION**

(43) Date of publication:
**11.07.2012 Bulletin 2012/28**

(51) Int Cl.:
***C09K 5/14*** *(2006.01)*

(21) Application number: **11160407.0**

(22) Date of filing: **30.03.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.01.2011 JP 2011001070**

(71) Applicant: **Firbest Co. Ltd.
Tokyo 103-0014 (JP)**

(72) Inventor: **Kikuta, Shunichi
Tokyo 103-0014 (JP)**

(74) Representative: **Viering, Jentschura & Partner
Grillparzerstrasse 14
81675 München (DE)**

(54) **Heat regenerating element and heat regenerating material using same**

(57)    There is provided a novel heat regenerating element having an excellent heat regenerating property which can accumulate thermal energy and effectively radiate the accumulated thermal energy to various objects. The present invention further provides a heat regenerating material including the heat regenerating element. The heat regenerating element of the present invention includes: 100 parts by weight of a base component composed of 60 to 90 parts by weight of a hydrotalcite compound and a remnant including at least one of zinc oxide and electrically-conductive zinc oxide; and 0.5 to 1 parts by weight of at least one heat regeneration enhancing component selected from a group of zirconium oxide components and zirconium carbide components.

## FIG. 1

EP 2 474 588 A2

## Description

### Cross Reference to Related Application

The priority application Japanese Patent Application No. 2011-001070 upon which this patent application is based is hereby incorporated by reference.

### Background of the Invention

### Field of the Invention

The present invention relates to a heat regenerating element which accumulates thermal energy and effectively radiates the accumulated thermal energy to a variety of objects, especially the heat regenerating element which is novel and having an excellent heat regenerating property. The present invention further relates to a heat regenerating material including the above-described heat regenerating element.

### Description of the Related Art

Conventionally, various materials including, for example, ceramics such as alumina, titania, zirconia or silica are used as a heat regenerating element. By including these heat regenerating elements, a heat regenerating material can be provided with an improved heat energy absorption efficiency and heat energy radiation efficiency. Such heat regenerating material can be used for heating, cooling and drying of an object and has been utilized in various applications such as a heating and cooling system and a medical care application.

For an efficient use of the heat energy with respect to an object such as a material or a human body by utilizing the absorption and radiation of the heat energy, both of the heat absorption and the heat radiation need to be performed efficiently. However, for the conventional heat regenerating elements mentioned above, both of the heat absorption efficiency and the heat radiation efficiency are not sufficient.

In Japanese Patent No. 2137667, the inventor of the present invention discloses a far-infrared radiating material which can efficiently radiate the heat energy required to excite a water molecule included in an object such as a human body. However, there is still a demand for a further improvement in the heat accumulation efficiency in the far-infrared radiating material described above.

If a heat energy source is a worm-blooded creature such as a human body, since energy of a far-infrared ray radiated from the worm-blooded creature is in wavelength of from about 2.5 to about 25 micrometers (typically within infrared range), there is required a heat regenerating element which can perform a high infrared absorption within this energy range.

In this regard, Japanese Patent Application Publication No. 2001-002408 discloses a method for improving a heat regenerating property by providing an anion which can absorb an infrared ray between crystal layers of a hydrotalcite.

However, the heat regenerating property of the heat regenerating material including the above-described hydrotalcite as the heat regenerating element is still not sufficient. Thus, there is a strong demand for a further improvement in the heat regenerating property of the heat regenerating element.

### Summary of the Invention

In view of the above-described problems, an object of the present invention is to provide a heat regenerating element which accumulates thermal energy and effectively radiates the accumulated thermal energy to a variety of objects, especially the heat regenerating element which is novel and having an excellent heat regenerating property.

Another object of the present invention is to provide a heat regenerating material including the above-described heat regenerating element.

In order to achieve the above-mentioned objects, the present invention provides a heat regenerating element including: 100 parts by weight of a base component, wherein the base component is composed of 60 to 90 parts by weight of a hydrotalcite compound and a remnant including at least one of zinc oxide and electrically-conductive zinc oxide; and 0.5 to 1 parts by weight of at least one heat regeneration enhancing component selected from a group of zirconium oxide components and zirconium carbide components.

Furthermore, the present invention provides a heat regenerating material including the above-mentioned heat regenerating element mixed with synthetic resin.

Furthermore, the heat regenerating material may be formed into a plate-shaped material, a tubular material, a sheet material, a fiber-like material or a material consisting of fibers.

**Advantageous Effect of the Invention**

According to the present invention, the heat regenerating element of the present invention can efficiently accumulate the heat energy and efficiently radiate the accumulated heat energy to various objects. Furthermore, the heat regenerating element of the present invention can be produced at low cost. Consequently, the heat regenerating element can be utilized in a wide variety of applications including drying and processing of various objects such as food, thermal insulation of farm and marine products, production of a fiber and such having the heat accumulation and thermal insulation effects and production of a heating and cooling system with improved efficiency.

Furthermore, according to the present invention, since the heat regenerating material is made of synthetic resin including the above-described heat regenerating element, the heat regenerating material is provided with a high heat regenerating effectiveness and can be formed into various shapes.

**Brief Description of the Drawing**

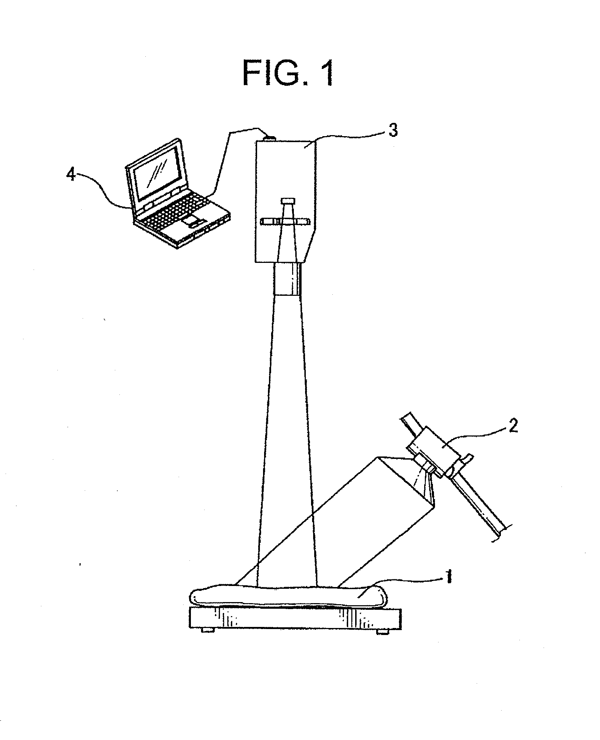

Fig. 1 is an illustration explaining a thermographic evaluation method.

**Detailed Description of the Invention**

The present invention will be described below in detail. As described above, a heat regenerating element of the present invention includes: 100 parts by weight of a base component and 0.5 to 1 parts by weight of at least one heat regeneration enhancing component. The base component is composed of 60 to 90 parts by weight of a hydrotalcite compound and a remnant including at least one of zinc oxide and electrically-conductive zinc oxide. The heat regeneration enhancing component is selected from a group of zirconium oxide and zirconium carbide.

The hydrotalcite compound described herein is defined as double hydroxide expressed by a general formula:

$$[Ma^{2+}_{1-x}Mb^{3+}_{x}(OH)_2]^{X+}[A^{n-}_{x/n} \cdot y\,H_2O]$$

wherein $Ma^{2+}$ is a divalent metal ion such as $Mg^{2+}$, $Co^{2+}$, $Ni^{2+}$ and $Zn^{2+}$, $Mb^{3+}$ is a trivalent metal ion such as $Al^{3+}$, $Fe^{3+}$ and $Cr^{3+}$, and $A^{n-}$ is a n-valent anion such as $OH^-$, $Cl^-$, $Co_3^{2-}$ and $SO_4^{2-}$. In general, x and y fall within a range of $0<x<0.33$, $0 \leq y<3$, respectively (refer to Bulletin of the Chemical Society of Japan, 1995(8), 622-628).

A representative example of the hydrotalcite compound is $Mg_{4.5}Al_2(OH)_{13}CO_3 \cdot 3.5H_2O$.

In addition, the hydrotalcite compound may be, for example, STABIACE HT-7 ($Mg_{3.5}Zn_{0.5}Al_2(OH)_{12}CO_3 \cdot 3H_2O$) and STABIACE HT-P ($Mg_{4.5}Al_2(OH)_{13}CO_3 \cdot 3.5H_2O$) both commercially available from Sakai Chemical Industry Co., Ltd., and DHT-7A ($Mg_{4.5}Al_2(OH)_{12.6}CO_3 \cdot 3.5H_2O$) and DHT-4A ($Mg_3ZnAl_2(OH)_{12}CO_3 \cdot 3H_2O$) both available from Kyowa Chemical Industry Co., Ltd..

In addition, the hydrotalcite having anion such as chloride ion between layers of the hydrotalcite (refer to Japanese Patent Application Publication No. 2001-002408) may also be used as the hydrotalcite compound.

The base component includes, in addition to the hydrotalcite compound, zinc oxide and/or electrically-conductive zinc oxide. The electrically-conductive zinc oxide is the zinc oxide as an n-type semiconductor created by doping a foreign atom into the zinc oxide. The electrically-conductive zinc oxide may be selected arbitrarily from a group of, but not limited to, gallium-doped zinc oxide, aluminum-doped zinc oxide, yttrium-doped zinc oxide, indium-doped zinc oxide, scandium-doped zinc oxide, titanium-doped zinc oxide, zirconium-doped zinc oxide and hafnium-doped zinc oxide, either singularly or in combination.

The above-described 100 parts by weight of the base component includes about 60 to 90 parts by weight of the hydrotalcite compound and the remnant consisting of the zinc oxide and/or the electrically-conductive zinc oxide. If the amount of the hydrotalcite compound in the base component is more than 90 parts by weight, the heat regenerating element cannot be dispersed efficiently in the synthetic resin during the manufacture of the heat regenerating material, resulting in a decrease in the heat absorption performance. On the other hand, if the amount of the hydrotalcite compound in the base component is less than 60 parts by weight, then the base component may be clumped together during the manufacture of the heat regenerating material, resulting in a significant decrease in the heat absorption performance.

The heat regenerating element is produced by mixing 0.5 to 1.0 parts by weight of the heat regeneration enhancing component with 100 parts by weight of the base component described above. The heat regeneration enhancing component may be zirconium oxide and/or zirconium carbide.

If the amount of the heat regeneration enhancing component is more than 0.5 parts by weight with respect to 100 parts by weight of the base component, the improvement in the heat regeneration efficiency is obtained. On the other hand, if the amount of the heat regeneration enhancing component is less than 0.5 parts by weight with respect to 100 parts by weight of the base component, there is no sufficient improvement in the heat regeneration efficiency. Furthermore, if the amount of the heat regeneration enhancing component is more than 1.0 parts by weight, there is no increase in the improvement in the heat regeneration efficiency which is comparable to the increase in the amount, thus wastefully increasing the manufacturing cost. The zirconium oxide may be stabilized by chemical combination with other components such as, but not limited to, $Y_2O_3$, CaO and MgO.

The above-described heat regenerating element may be a mixture of the base component and the heat regeneration enhancing component both in form of powder. Alternatively, it is possible to mix some of or all of the above-described components first, followed by sintering the mixture at a high temperature and then pulverizing the sintered mixture using a pulverizing means such as a ball mill. The respective components of the heat regenerating element obtained has a diameter suitable to be dispersed uniformly in the synthetic resin when the respective components are mixed in the synthetic resin to form the desirable heat regenerating material of various kinds. Generally, the diameter of the respective components is equal or less than 1 micrometer, and preferably equal or less than 0.5 micrometers.

The heat regenerating material is obtained from the above-described heat regenerating element of the present invention and the synthetic resin of various kinds.

For example, the heat regenerating element and the synthetic resin may be mixed together and subjected to kneading, or the heat regenerating element may be added to the molten synthetic resin and then pelletized using extrusion molding as needed to obtain a mixture including the heat regenerating element dispersed uniformly in the synthetic resin. Subsequently, the mixture of the heat regenerating element and the synthetic resin is processed to produce a heat regenerating material in form of a sheet (or film), a plate-like shape, a tubular shape, a fiber or into a variety of other forms or shapes, by using molding method such as injection molding, extrusion molding, T-die molding and calendaring or by using spinning method such as melt spinning and centrifugal spinning. In addition, the obtained heat regenerating material may be subjected to a secondary process. For example, the heat regenerating material in form of fibers may be subjected to the secondary process to form a material consisting of the fibers such as a paper (may or may not include thermal adhesive composite fibers from Chisso Co.), a felt, a perforated sheet, an unwoven fabric or woven fabric (may be of three-dimensional).

The preferable synthetic resin used to produce the heat regenerating material may be, but not limited to, olefinic polypropylene or polyethylene or acrylic synthetic resin or the like.

Alternatively, the heat regenerating element of the present invention may be added for example to a binder of the synthetic resin to be used as a coating material.

The following will describe some exemplary embodiments of the present invention.

In a first exemplary embodiment, hydrotalcite compound powder (STABIACE HT-P from Sakai Chemical Industry Co., Ltd., average particle diameter of 0.45 micrometers), zinc oxide powder (average particle diameter of 0.2 micrometers), conductive zinc oxide (CZnO) powder (23-K from Hakusui Tech Co., Ltd., aluminum-doped, average particle diameter of 0.2 micrometers), zirconium oxide powder (average particle diameter of 0.2 micrometers) and zirconium carbide powder (average particle diameter of 0.3 micrometers) are mixed according to the composition amount (parts by weight) shown in Table 1 to obtain the heat regenerating elements A through H. In addition, for a comparison purpose, comparative heat regenerating elements I through X are obtained from the same feedstock components listed above but with different composition amount, as shown in Table 2 and Table 3.

Table 1

| feedstock | A | B | C | D | E | F | G | H |
|---|---|---|---|---|---|---|---|---|
| HT | 90 | 70 | 70 | 60 | 90 | 70 | 70 | 60 |
| ZnO | 10 | 30 | 30 | 40 | | | | |
| CZnO | | | | | 10 | 30 | 30 | 40 |
| $ZrO_2$ | 0.5 | 0.5 | 1 | 0.5 | | | | |
| ZrC | | | | | 1 | 1 | 0.5 | 1 |
| T(°C) | 52.7 | 50.9 | 51. 6 | 51.8 | 50.1 | 50.6 | 50.8 | 51.2 |
| Δ T (°C) | 5.7 | 3.9 | 4.6 | 4.8 | 3.1 | 3.6 | 3. 8 | 4.2 |

Table 2

| feedstock | I | J | K | L | M | N | O | P |
|---|---|---|---|---|---|---|---|---|
| HT | 95 | 40 | 95 | 40 | 100 | | | 70 |
| Zn0 | 5 | 60 | | | | 100 | | 30 |
| CZn0 | | | 5 | 60 | | | 100 | |
| ZrO$_2$ | 2 | 2 | | | | | | 0.1 |
| ZrC | | | 2 | 2 | | | | |
| T (°c) | 49.2 | 48.4 | 47.5 | 47.7 | 47.0 | 46.8 | 47.2 | 49.3 |
| A T(°C) | 2.2 | 1.4 | 0.5 | 0,7 | 0,0 | -0.2 | 0.2 | 2.3 |

Table 3

| feedstock | Q | R | S | T | U | V | W | X | α |
|---|---|---|---|---|---|---|---|---|---|
| H T | 60 | 90 | 70 | 60 | 5 | 5 | 50 | 50 | |
| Zn0 | 40 | | | | 9 | | 50 | | |
| CZnO | | 10 | 30 | 40 | | 95 | | 50 | |
| ZrO$_2$ | 0.1 | | | | 0.5 | | 5 | | |
| ZrC | | 0.1 | 0.1 | 0.1 | | 0.5 | | 5 | |
| T (°C) | 49.1 | 49.0 | 48.6 | 48. 8 | 47.9 | 47.3 | 48.2 | 49.2 | 47.0 |
| ΔT (°C) | 2.11 | 2.0 | 1. 6 | 1.8 | 0.9 | 0.3 | 1.8 | 2.2 | - |

Then, 1 part by weight of the respective heat regeneration materials A through X are mixed with 100 parts by weight of the polypropylene resin, respectively, using a Henschel mixer at the rotating speed of 500 rpm for 4 minutes. Then, the respective mixtures are subjected to extrusion molding at a temperature of 220 degrees Celsius by using a single screw extruder with $\phi$ =50 mm (NS-50 from Nippon Placon Co., Ltd.). The extruded mixture is then cooled and cut to produce total of 24 kinds of polypropylene resin composition pellets. The uniformity of the particle dispersion of the heat regenerating element in the synthetic resin was evaluated, and it was observed visually that the particle dispersion of the heat regenerating element was uniform for all of the resin composition pellets.

Furthermore, a control material including no heat regenerating element (indicated by "α" in Fig. 3) was also prepared in form of a pellet for a comparison purpose.

Next, the above-mentioned 24 kinds of polypropylene resin compositions and the control material were respectively subjected to the melt-spinning process and are subsequently elongated using an air sucker to obtain a fiber having fineness of 3 decitexes.

The obtained fibers are processed using a roller carding machine (M32S-5000R from Ikegami Kikai Co., Ltd.) to produce a web. This web is then processed by a needle punching machine (NL-600 from Ikegami Kikai Co., Ltd.) to obtain an unwoven fabric (i.e. the heat regenerating material in form of the unwoven fabric). The obtained unwoven fabric is 500mm in width, 2mm in thickness and 100g/m$^2$ in weight per unit area. The color of the respective unwoven fabrics obtained from the respective kinds of heat regenerating element is substantially white, and there was no visually significant difference in the appearance and color between the respective unwoven fabrics.

Next, an unwoven fabric test piece (100mm x 160mm in size) was cut out from the respective unwoven fabrics obtained above and was evaluated according to an evaluation method shown in Fig. 1. In Fig. 1, a halogen lamp 2 of 250W (a floodlight unit from CUSTOM KOBO Co.) is arranged at 45 degrees obliquely upward of and 30cm apart from the unwoven fabric test piece 1, and a thermographic device 3 (a thermo tracer TH9100MR from NEC Avio Infrared Technologies Co., Ltd.) is arranged at vertically upwards of and 80cm apart from the unwoven fabric test piece 1. In this method, the unwoven fabric test piece 1 was irradiated with the halogen lamp 2, and after 180 seconds of irradiation, an average temperature of central area (65mm x 85mm) of the unwoven fabric test piece 1 was measured. For a control unwoven fabric piece obtained from the control material α which does not include the heat regenerating element, the average temperature of the central area after 180 seconds of irradiation measured was 47.0 degrees Celsius. For each of the 24 kinds of unwoven fabric test pieces, the temperature difference (ΔT) between the average temperature of the respective unwoven fabric test pieces and the average temperature of the control unwoven fabric piece was calculated by a calculator 4 connected to the thermographic device 3. The results are shown in Fig. 1 though Fig. 3.

This evaluation method employs spectral emissivity evaluation criteria for the far-infrared region defined by Japan Far Infrared Rays Association for discussing the results for the temperature difference $\Delta T$. According to this evaluation criteria, when the total emissivity for the measurement area of the unwoven fabric test piece is 5% above (greater than) the total emissivity for the measurement area of the control piece, then the difference is found to be significant. Thus, based on this method, it can be found that if the average temperature of the unwoven fabric test piece was 5 % greater than the average temperature of the control unwoven fabric piece, then the temperature difference $\Delta T$ between the average temperatures of the unwoven fabric test piece and the control unwoven fabric piece is significant, that is, the heat regenerating element is determined as effective in the heat generation. Thus, it can be found that the unwoven fabric test piece is effective in heat generation if the average temperature of the unwoven fabric test piece is above 49.5 degrees Celsius, which is 5 % above the average temperature of the control unwoven fabric piece which is 47.0 degrees Celsius.

Therefore, from the results shown in the results shown in Fig. 1 through Fig. 3, the improvement in the heat generation was observed only for the unwoven fabrics including the heat regenerating element according to the present invention (the unwoven fabrics obtained from the heat regenerating elements A through H).

Furthermore, there is prepared another unwoven fabric which is obtained from the heat regenerating element based on the composition of the heat regenerating element M shown in Fig. 3, but including the hydrotalcite compound powder (DHT-4A from Kyowa Chemical Industry Co., Ltd. and particle diameter of 0.7 micrometers) having chloride ion (C1-) between crystal layers. Then this unwoven fabric was evaluated according to the above-described evaluation method. The result shows that there was no significant improvement observed in the heat generation. However, for yet another unwoven fabric prepared based on the heat regenerating element H but replacing STABIACE HT-P from Sakai Chemical Industry Co., Ltd. with DHT-4A, the result of the evaluation shows that this unwoven fabric exhibits the heat generation effect of the same level as the unwoven fabric obtained from the heat regenerating element H.

Furthermore, there is prepared yet another unwoven fabric obtained based on the heat regenerating element H but replacing STABIACE HT-P with DHT-7A. The result of the evaluation again shows that this unwoven fabric exhibits the heat generation effect of the same level as the unwoven fabric obtained from the heat regenerating element H.

A second exemplary embodiment of the present invention relates to an evaluation of drying of a beef jerky in a reduced-pressure drying unit. In this exemplary embodiment, firstly, stock solution including acrylonitrile (AN), vinyl acetate and sodium ethenesulfonate at molar ratio of 94.5:5.1:0.4 is prepared and is polymerized in DMSO (dimethylsulfoxide) solvent. Also, slurry was prepared separately by uniformly dispersing the heat regenerating element E in DMSO. Then, the stock solution is added to the slurry to produce spinning stock solution having an amount ratio of the heat regenerating element E:DMSO:the stock solution corresponding to 10:87:3. This spinning stock solution includes 100 parts by weight of polymer component and 2 parts by weight of the heat regenerating element E.

The obtained spinning stock solution is discharged from a spinning nozzle into DMSO solution having a temperature of 35 degrees Celsius and concentration of 65%, elongated to 4.0 to 4.5 times in size, washed with warm water at 60 degrees Celsius to remove the solvent in the fiber, and finally subjected to drying and heating at 160 degrees Celsius to perform densification while maintaining the shrinkage ratio within 0 to 3%. As a result, a heat insulating fiber EA (heat regenerating material in form of a fiber) having fineness of 2.2 decitexes is obtained.

Next, 80 percent by weight of the above-described heat insulating fiber EA, 15 percent by weight of polyester fiber having fineness of 2.2 decitexes and 5 percent by weight of thermal-adhesive fiber are mixed together and subjected to confounding using the needle punching machine. Then, using thermals rolls, the fibers are pressure-bonded in which fibers having low melting point are softened and melted to be thermally adhered each other. Finally, there is obtained a heat regenerating material sheet EAS having a size of 1m$^2$. The heat regenerating material sheet EAS is 2mm in thickness and 200g/m$^2$ in weight per unit area and area. The series of processes described above correspond to a needle punching and thermal bonding method which is a general method of manufacturing unwoven fabric.

For a comparison purpose, a control sheet OAS is also prepared in a similar manner as described above, except the heat regenerating element O is used instead of the heat regenerating element E.

The respective heat regenerating material sheet EAS and the control OAS sheet are arranged at inside of respective gas indirect heating blower exhaust-type reduced-pressure drying device so as to be attached to an upper wall and the side walls of the reduced-pressure drying device via heat-resistant adhesion tape. Each of the reduced-pressure drying devices has internal width of 1.5m, internal length of 3.5m and internal height of 2.1m.

Lumps of beef jerky each having 10mm in diameter, 150mm in length and 25g in weight are arranged on a 6500mm$^2$ tray so as to be 50mm in height (thus total of 5kg). 15 trays are stacked to a wagon and 6 wagons (thus total of 450kg) are placed in the respective reduced-pressure drying device.

For each of the reduced-pressure drying devices, the reduced-pressure drying experiment is performed at a temperature of 68 degrees Celsius and in a vacuum of 0.02 MPa, and the drying time (minutes) required to acquire the moisture percentage of 26%±0.5% was measured. This reduced-pressure drying experiment is performed on the respective reduced-pressure drying devices for 10 times each between May 28 and June 14. The results are shown in Fig. 4.

The moisture percentage is measured automatically using a infrared moisture meter (FD-610 from Kett Electric Laboratory, based on heat drying and weight measurement).

Table 4

| date | | 5/28 | 6/1 | 6/1 | 6/2 | 6/4 | 6/7 | 6/7 | 6/9 | 6/10 | 6/14 | average |
|------|-----|------|-----|-----|-----|-----|-----|-----|-----|------|------|---------|
| sheet | EAS | 160 | 170 | 230 | 150 | 170 | 170 | 240 | 160 | 240 | 160 | 185 |
| | OAS | 190 | 180 | 260 | 170 | 180 | 180 | 260 | 180 | 270 | 180 | 205 |

As shown in Fig. 4, it is observed that the time required to acquire the above-described moisture percentage was shorter for the reduced-pressure drying device provided with the heat regenerating material sheet EAS using the heat regenerating element E according to the present invention than for the reduced-pressure drying device provided with the control sheet OAS.

The embodiments described herein are only representative embodiments and are not intended to limit the present invention. It will be understood that various modifications to the embodiments may be made without departing the frame of the present invention.

**Claims**

1. A heat regenerating element comprising:

   100 parts by weight of a base component, wherein the base component is composed of 60 to 90 parts by weight of a hydrotalcite compound and a remnant including at least one of zinc oxide and electrically-conductive zinc oxide; and
   0.5 to 1 parts by weight of at least one heat regeneration enhancing component selected from a group of zirconium oxide components and zirconium carbide components.

2. A heat regenerating material comprising the heat regenerating element described in claim 1 mixed with synthetic resin.

3. The heat regenerating material described in claim 2, wherein the heat regenerating material is formed into a plate-shaped material, a tubular material, a sheet material, a fiber-like material or a material consisting of fibers.

# FIG. 1

## REFERENCES CITED IN THE DESCRIPTION

**Patent documents cited in the description**

- JP 2011001070 A
- JP 2137667 A
- JP 2001002408 A